# EUROPEAN PATENT APPLICATION

(11) **EP 3 235 474 A1**
(43) Date of publication of application: **25.10.2017**
(21) Application number: 16165737.4
(22) Date of filing: 18.04.2016
(51) Int. Cl.: A61F 5/37, A61F 5/01

(54) **SHOULDER BRACE**

(71) Applicant: Roessingh Beheer B.V., 7522 AH Enschede (NL)
(72) Inventor: de Koning, John-John, 7545 XL Enschede (NL); van Vliet, Reinout Otto, 7524 BG Lonneker (NL)
(74) Representative: 't Jong, Bastiaan Jacob

(57) **Abstract**

The invention relates to a shoulder brace for approximation of the humerus head in the shoulder joint of a person, which shoulder brace comprises:
- a shoulder cap adapted to be positioned on top of a shoulder of a person;
- an adjustable armpit belt, wherein both ends of the adjustable armpit belt are arranged to the shoulder cap, which adjustable armpit belt is adapted to run along the armpit opposite of the shoulder on top of which the shoulder cap is positioned;
- a upper arm sleeve adapted to be worn around the upper arm of a person;
- at least one adjustable strap arranged with one end to the shoulder cap and with the other end to the upper arm sleeve.

## Description

The invention relates to a shoulder brace for approximation and adequate positioning of the humerus head in the shoulder of a person. Good positioning of the humerus head according to the joint surface will prevent or reduce the anterior and/or caudal (sub)luxation of humerus head.

This subluxation in most of the cases due to dysbalance of shoulder stabilizing muscles as a result of central neurological causes (like stroke), plexopathies, neuromuscular disorders or serious hypermobility syndromes (like Ehlers Danlos Syndrome). Traction of the shoulder structures caused by the weight and movements of the arm causes pain by local elongation of the joint capsule, microdamage of the glenohumeral joint or overload of residual stabilizing rotator cuff muscles because of overcompensation

A commonly known manner of relieving the shoulder is by use of a sling. The sling is wrapped around the forearm and around the neck of the person. As a result the weight of the arm is borne by the neck and the arm is immobilized against the body. The neck is however not suited for carrying such a weight and after a day wearing a sling, the muscles in the neck will start to ache. Also, the fact that one of the arms cannot be properly used, as it is immobilized, will result in overloading other parts of the body, such as the other arm and the back.

Improved slings are known in the prior art, in which the sling does not run over the neck, but runs over the shoulder. This relieves the neck, but the forearm is still immobilized by the sling being wrapped around it.

Another shoulder brace known in the prior art has a stocking like sleeve, which is worn around the upper arm and over the shoulder. Elastic straps are arranged on this sleeve and run along the back and neck of the person to keep the sleeve into place. However, as the sleeve is to be put on like a stocking, the sleeve also needs to be elastic and any grip on the upper arm is only achieved by the elastic force and friction of the sleeve. The elastic force needed to get grip on the upper arm may cause blood circulation problems. Also due change of anatomic structures as muscle tissue a standard sleeve dos not fit well and approximation of the shoulder will be lost.

So, the known shoulder braces either provide insufficient support, such that the shoulder is not relieved sufficiently and might cause blood circulation problems, or the known shoulder braces provide sufficient support, while immobilizing the full arm.

It is accordingly an object of the invention to reduce or even remove the above mentioned disadvantages of the prior art.

This object is achieved according to the invention with a shoulder brace comprising:
- a shoulder pad adapted to be positioned on top of a shoulder of a person;
- an adjustable armpit belt, wherein both ends of the adjustable armpit belt are arranged to the shoulder pad, which adjustable armpit belt is adapted to run along the armpit opposite of the shoulder on top of which the shoulder pad is positioned;
- an upper arm sleeve adapted to be worn around the upper arm of a person;
- at least one adjustable strap arranged with one end to the shoulder pad and with the other end to the upper arm sleeve.

The shoulder brace according to the invention comprises two mounting points, i.e. the shoulder pad and the upper arm sleeve. The distance between these mounting points can be adjusted by the at least one adjustable strap, such that independent at which exact position the upper arm sleeve is arranged on the upper arm, the upper arm sleeve can always be pulled up towards the shoulder pad to approximate the humerus head in de shoulder.

The adjustable armpit belt prevents that the shoulder pad does not slide of the shoulder in time and the approximating action is maintained. This ensures the working mechanism of the brace.

As the shoulder brace is only positioned between the upper arm and shoulder, the forearm is free to move within the range of motion (ROM) of a person with a disability. ( The wearer wears the shoulder brace without any restriction of the shoulder brace it's self.).

In a preferred embodiment of the shoulder brace according to the invention the upper arm sleeve is lined with a high friction material, such as polyurethane, to provide grip on the skin of the upper arm.

High friction materials are known in the prosthetic field and provide a good grip on the skin without causing substantial irritation of the skin. By arranging a liner of this high friction material on the upper arm sleeve, the upper arm sleeve can get an improved grip on the upper arm without disturbing blood circulation due to the non circular tension of the upper arm sleeve, which contributes to approximate the humerus head. It is very preferred to use this material. Other materials (e.g. foam) does not have this combination of characteristics. (friction, minimal irritation of the skin) Preferably polyurethane (PU) is used. This material has the characteristics of high friction, minimal skin irritation, minimal deformation.

In another embodiment of the shoulder brace according to the invention the shoulder pad is also lined with a high friction material to provide grip on the skin of the shoulder.

By arranging a liner of high friction material to the shoulder pad, the shoulder pad can maintain its position more easily due to the improved friction. This allows for the tension in the arm pit belt to be reduced, contributing to improved comfort for the wearer of the shoulder brace.

In a further preferred embodiment of the shoulder brace according to the invention the at least one adjustable strap is provided with a boa closure system to adjust the length of the strap.

A boa closure system is a known type of closure. The boa closure system has stainless steel wires running through low friction lace guides and terminating in a reel to replace regular laces. The reel has a friction lock, such that the reel can be turned to wind up the steel wires or the wind off the wires, such that the length of the closure system is adjusted and accordingly the length of the adjustable strap.

The advantage of the boa closure system is that a person, wearing the shoulder brace can easily with minimal force and very precisely adjust the adjustable strap by turning with one hand the reel of the boa system. This turning of the reel requires only limited function of a hand, such that disabled people can also easily adjust the straps.

In a further preferred embodiment of the shoulder brace according to the invention two adjustable straps are each arranged with one end to the shoulder pad and with the other end to the upper arm sleeve, wherein each adjustable strap is provided with a boa closure system, and wherein each boa closure system is arranged near the shoulder pad.

By providing two adjustable straps, the position of the upper arm sleeve relative to the shoulder pad can be adjusted. As a result, the upper arm can be urged more to the front or to the back by adjusting the length and accordingly the tension of the two adjustable straps. Hereby, the person wearing the shoulder brace can set such a tension in the shoulder brace, that any pain in the shoulder is optimally reduced.

In still a further embodiment of the shoulder brace according to the invention the attachment of the one end of the at least one adjustable strap to the humerus head upper arm sleeve is provided with a hook and loop attachment. This allows for the needed force vector lines to be achieved. This results in the optimum positioning of humerus head.

The hook and loop attachment, also known as Velcro, allows for easy detachment of the upper arm sleeve from the adjustable straps. So, a person can put on the sleeve and the shoulder pad separately, then attach the adjustable strap to the sleeve and adjust the strap to the correct length, such that the shoulder is relieved.

These and other features of the invention will be elucidated in conjunction with the accompanying drawings.
Figure 1 shows a front view of an embodiment of the shoulder brace according to the invention.

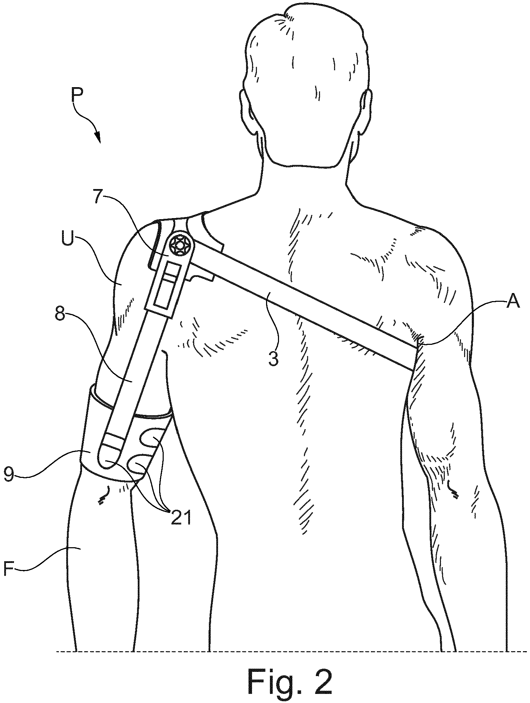

Figure 2 shows a rear view of an embodiment of the shoulder brace according to the invention.
Figures 3A and 3B show cross-sectional views of part of the embodiment according to figure 1.

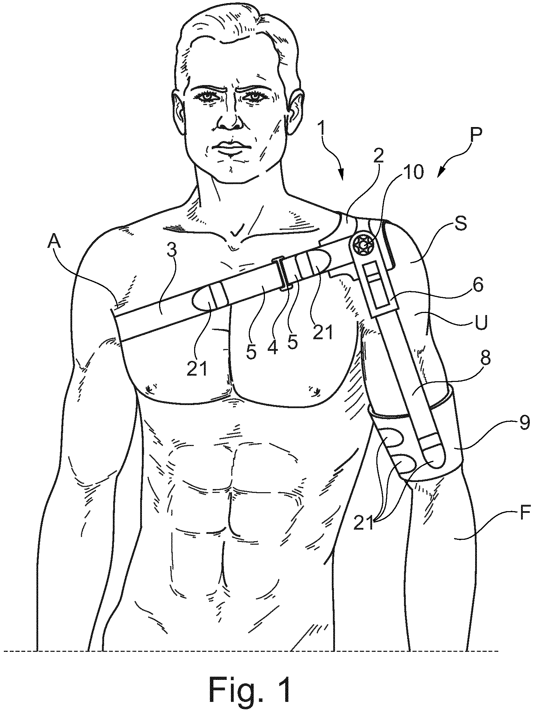

Figure 1 shows a person P having a left shoulder S, a left upper arm U and a left forearm F, as well as a right armpit A.

The person P wears a shoulder brace 1 with a shoulder pad 2 to which an armpit belt 3 is arranged. This armpit belt 3 runs from the shoulder pad 2 over the breast, under the armpit A and over the back in a loop to the shoulder pad 2. The armpit belt 3 has a loop 4 and a free end 5 wrapped around the loop 4 and attached by for example a hook and loop attachment. This allows for adjustment of the armpit belt 3.

Two boa closure systems 6, 7 are attached to the shoulder pad 2 to provide an adjustable strap 8, which is attached to an upper arm sleeve 9. The boa closure system 6 can be adjusted by rotating the knob 10.

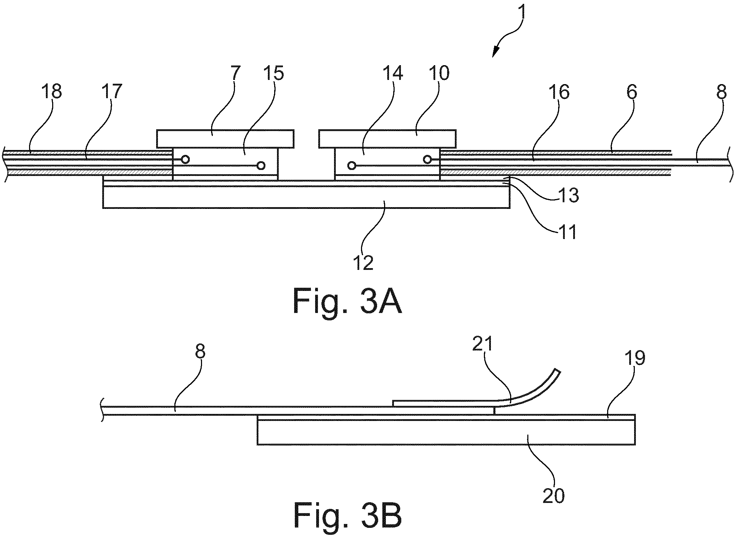

Figure 3A shows a cross sectional view of the shoulder pad section of the shoulder brace 1. The shoulder pad has a loop top layer 11 and a high friction material layer 12 for contact with the skin of the person P.

The two boa closure systems 6, 7 are arranged on the top layer 11 with self adhesive hook 13 witch is stuck to the back of each boa closure system. Each boa closure system 6, 7 has a housing 14, 15 with a reel inside, which is for winding up or unwinding a steel cable 16, 17. Each reel is connected to a knob 10, 7, such that the person P can easily adjust the length of the cable 16, 17 and accordingly the length of the strap 8, which is attached to the cable 16. The strap 8 is guided by a channel 6, 18 attached to the housing 14, 15 of the boa closure system 6, 7.

Figure 3B shows a cross-sectional view of the upper arm sleeve, which has also a top layer 19 and a layer of high friction material 20. The top layer 19 is provided with loops, such that the end of the strap 8, which is provided with hooks can easily be attached to the upper arm sleeve 19, 20.

The end of the strap 8 is furthermore provided with a tab 21, which facilitates for the person P to grab the end of the strap 8 and to tear the strap 8 loose from the upper arm sleeve.

## Claims

1. Shoulder brace for approximation of the humerus head into the shoulder joint of a person, which shoulder brace comprises:
- a shoulder pad adapted to be positioned on top of a shoulder of a person;
- an adjustable armpit belt, wherein both ends of the adjustable armpit belt are arranged to the shoulder pad, which adjustable armpit belt is adapted to run along the armpit opposite of the shoulder on top of which the shoulder pad is positioned;
- an upper arm sleeve adapted to be worn around the upper arm of a person;
- at least one adjustable strap arranged with one end to the shoulder pad and with the other end to the upper arm sleeve.

2. Shoulder brace according to claim 1, wherein the upper arm cuff is lined with a high friction material to provide grip on the skin of the upper arm.

3. Shoulder brace according to claim 1 or 2, wherein the shoulder cap is lined with a high friction material, such as polyurethane, to provide grip on the skin of the shoulder.

4. Shoulder brace according to any of the preceding claims, wherein the at least one adjustable strap is provided with a boa closure system to adjust the length of the strap and thereby adjust the traction forces.

5. Shoulder brace according to claim 4, wherein two adjustable straps are each arranged with one end to the shoulder cap and with the other end to the upper arm sleeve, wherein each adjustable strap is provided with a boa closure system, and wherein each boa closure system is arranged near the shoulder cap.

6. Shoulder brace according to any of the preceding claims, wherein the attachment of the one end of the at least one adjustable strap to the upper arm sleeve is provided with a hook and loop attachment.
